# EUROPÄISCHE PATENTSCHRIFT

(11) **EP 4 144 385 B1**
(45) Veröffentlichungstag und Bekanntmachung des Hinweises auf die Patenterteilung: **17.07.2024**
(21) Anmeldenummer: 21194257.8
(22) Anmeldetag: 01.09.2021
(51) Int. Cl.: A61L 9/12, B60H 3/00, A61L 9/04, B60H 1/00

(54) **BEDUFTUNGSVORRICHTUNG FÜR EIN FAHRZEUG**
AIR FRESHENER DEVICE FOR A VEHICLE
DISPOSITIF DE DIFFUSION DE PARFUM DANS UN VÉHICULE

(43) Veröffentlichungstag der Anmeldung: 08.03.2023
(73) Patentinhaber: Supair-Tel AG, 8152 Glattbrugg (CH)
(72) Erfinder: Guggenheim, Rudolf Heinrich, 8832 Wollerau (CH)
(74) Vertreter: E. Blum & Co. AG

(56) Entgegenhaltungen:
- US-A1- 2020 331 326
- US-B1- 11 059 353

## Beschreibung

### Gebiet der Erfindung

Die Erfindung betrifft eine Beduftungsvorrichtung für ein Fahrzeug mit einem Gehäuse und einem im Gehäuse angeordneten Duftstoffbehälter. Im Weiteren umfasst die Beduftungsvorrichtung eine Haltevorrichtung zum Anbringen der Beduftungsvorrichtung an einer Lüftung im Innenraum eines Fahrzeuges. Die Haltevorrichtung weist ein Halteelement mit einer Öffnung auf. Die Öffnung weist eine Tiefe auf und ist in Form eines Durchbruchs oder einer Vertiefung ausgestaltet.

### Hintergrund

Es sind Beduftungsvorrichtungen bekannt, welche im Fahrzeuginneren aufgehängt werden können und z.B. ein mit Duftstoff getränktes Material aufweisen. Solche Vorrichtungen sind kostengünstig herstellbar, ihre Beduftungswirkung kann aber ungleichmässig sein und der in der Regel am Innenrückspiegel aufgehängte Duftkörper kann sichtbehindernd sein.

Im Weiteren sind Behälter mit Duftstoff bekannt, welche an einem Lüftungsgitter im Innenraum eines Fahrzeuges befestigt werden. Der Behälter für den Duftstoff weist Einlassöffnungen auf, sodass Luft aus der Lüftung in den Behälter hineinströmt, mit dem Duftstoff in Kontakt gerät und angereichert mit Duftmolekülen den Behälter durch Auslassöffnungen verlässt. Solche Behälter können mittels eines Klemmteils am Lüftungsgitter befestigt und von diesem auch wieder entfernt werden.

Dokument US 2020/331326 A1 beschreibt eine derartige Beduftungsvorrichtung mit einer Haltevorrichtung und einer Einlassöffnung. Dokument US 11 059 353 B1 offenbart eine weitere herkömmliche Beduftungsvorrichtung mit einem Duftstoffbehälter und einer Öffnung für die Aufnahme eines Halteelements.

### Darstellung der Erfindung

Es stellt sich die Aufgabe, eine Beduftungsvorrichtung bereitzustellen, welche zuverlässig an einer Lüftung eines Fahrzeuges montiert werden kann.

Diese Aufgabe wird durch eine Beduftungsvorrichtung für ein Fahrzeug mit den Merkmalen des Anspruchs 1 gelöst. Der Begriff "Fahrzeug" umfasst Landfahrzeuge, wie Personenkraftwagen und Lastkraftwagen, und auch Luftfahrzeuge.

Die erfindungsgemässe Beduftungsvorrichtung umfasst ein Gehäuse, in welchem ein Duftstoffbehälter angeordnet ist. Zudem umfasst die Beduftungsvorrichtung eine Haltevorrichtung zum Anbringen der Beduftungsvorrichtung an einer Lüftung im Innenraum des Fahrzeuges. Die Haltevorrichtung umfasst ein Halteelement, welches eine Öffnung oder ein Loch aufweist. Die Öffnung weist eine Tiefe auf und ist in Form eines Durchbruchs oder einer Vertiefung ausgestaltet.

Ist die Öffnung in Form eines Durchbruchs ausgestaltet, so ist die Öffnung durch das Halteelement durchgängig, d.h. die Tiefe der Öffnung entspricht der Dicke des Halteelements. Handelt es sich bei der Öffnung um eine Vertiefung, ist die Öffnung nicht durchgängig und die Öffnung ist nur einseitig zugänglich.

Am Rand bzw. an den Wänden der Öffnung sind Vorsprünge angeordnet. Diese Vorsprünge erstrecken sich vom Rand in das Innere der Öffnung. In Richtung der Tiefe erstrecken sich die Vorsprünge aber unterschiedlich weit in das Innere der Öffnung, insbesondere verjüngen sich die Vorsprünge in Richtung der Tiefe.

Vorsprünge, welche sich in Richtung der Tiefe unterschiedlich weit in das Innere der Öffnung erstrecken, haben den Vorteil, dass zwischen den Vorsprüngen unterschiedlich grosse Gegenstände optimal eingeklemmt werden können. Die Beduftungsvorrichtung kann somit zuverlässig auf unterschiedlich grosse Gegenstände geklemmt werden.

Das Halteelement kann insbesondere als Material Silikon, Gummi und/oder ein Elastomer, insbesondere ein thermoplastisches Elastomer, umfassen. Insbesondere kann es ein Material mit einer Härte im Bereich von 30 Shore-A bis 90 Shore-A umfassen.

Mit Vorteil sind der Durchbruch oder die Vertiefung in Form eines Zylinders ausgestaltet, insbesondere in Form eines Kreiszylinders oder in Form eines Zylinders mit einer elliptischen Grundfläche. Die zylindrische Form umfasst eine Mantelfläche und eine Höhe. Die Höhe der zylindrischen Form entspricht der Tiefe der Öffnung. Die Vorsprünge sind an der Mantelfläche angeordnet und erstrecken sich entlang der Höhe unterschiedlich weit in das Innere der zylindrischen Öffnung.

Zylindrische Formen mit einem Kreis oder eine Ellipse als Grundfläche erlauben, in der Haltevorrichtung runde oder Zylindrische Elemente, insbesondere Knöpfe, einzuklemmen. Die Beduftungsvorrichtung kann dadurch auf an der Lüftung angebrachte Knöpfe angeordnet werden.

Insbesondere sind die Vorsprünge hohl ausgebildet. Ein hohles Innere hat eine höhere Elastizität der Vorsprünge zur Folge. Die Vorsprünge können sich somit einfacher und flexibler an die Form einzuklemmender Elemente anpassen. Insbesondere ist der Hohlraum in der Form einer Vertiefung und nicht in der Form eines Durchbruchs ausgestaltet.

Mit Vorteil sind die einzelnen Vorsprünge voneinander beabstandet, d.h. die einzelnen Vorsprünge berühren sich nicht, insbesondere nicht am Rand der Öffnung. Der Abstand ist insbesondere grösser als ein Drittel der Breite des Vorsprungs. Ein Zwischenraum zwischen den Vorsprüngen hat den Vorteil, dass sich die Vorsprünge auch in diesen Zwischenraum elastisch verformen können. Dies hat zur Folge, dass sich die Vorsprünge besser an die Form einzuklemmender Elemente anpassen können und die Beduftungsvorrichtung zuverlässiger haften bleibt.

Vorteilhaft weist die Öffnung eine Tiefe bzw. eine Höhe von mindestens 5 mm, insbesondere von mindestens 7.5 mm auf. Solche Grössen gewährleisten eine genügend grosse Fläche innerhalb der Öffnung, mit welcher die Beduftungsvorrichtung an der Lüftung haften kann.

Insbesondere erstrecken sich die Vorsprünge entlang der Tiefe über mindestens 50%, insbesondere mindestens 75%.

In einer besonderen Ausführungsform weist die Haltevorrichtung eine Nut auf, welche die Öffnung umgibt. Insbesondere ist die Nut kreisförmig und insbesondere geeignet, die Haltevorrichtung am Gehäuse zu befestigen. Mit der vorgesehenen Nut kann die Haltevorrichtung in einfacher Weise auf das Gehäuse aufgespannt werden.

Insbesondere umfasst die Beduftungsvorrichtung eine Schiebevorrichtung mit welcher Einlass- und/oder Auslassöffnungen des Gehäuses mittels einer Schiebebewegung zumindest teilweise verschlossen werden können.

Mit Vorteil weist die Schiebevorrichtung mindestens zwei Griffe aufweist, durch dessen Betätigung die Schiebbevorrichtung verschiebbar ist, insbesondere wobei die Betätigung durch Hineindrücken der Griffe in das Gehäuse erfolgt. Die Griffe können derart ausgestaltet sein, dass durch Betätigung des einen der mindestens zwei Griffe die Einlass- und/oder Auslassöffnungen geöffnet und durch Betätigung eines anderen der mindestens zwei Griffe die Einlass- und/oder Auslassöffnungen verschlossen werden können.

Insbesondere weist die Schiebevorrichtung eine Arretiervorrichtung auf, derart dass in arretierter Stellung die Griffe nicht betätigbar und die Schiebebewegung nicht durchführbar sind. Dadurch sollen ungewollte Manipulationen an der Beduftungsvorrichtung verhindert werden.

In einer besonderen Ausführungsform umgibt der Duftstoffbehälter das Halteelement und/oder der Duftstoffbehälter weist eine Öffnung, insbesondere eine durchgängige Öffnung, auf, innerhalb welcher das Halteelement angeordnet ist. Insbesondere ist die Beduftungsvorrichtung ausgestaltet, genau einen Duftstoffbehälter im Gehäuse aufzunehmen.

Vorteilhaft weist die Beduftungsvorrichtung eine nicht durchgängige Vertiefung auf, welche geeignet ist, dass bei Anbringung der Beduftungsvorrichtung an der Lüftung ein Regelknopf der Lüftung in die Vertiefung greifen kann. Insbesondere ragen die Vorsprünge in die Vertiefung der Beduftungsvorrichtung hinein, sodass mittels den Vorsprüngen der Regelknopf innerhalb der Vertiefung eingeklemmt werden kann. Die Beduftungsvorrichtung haftet dadurch am Regelknopf und kann von der Lüftung angeströmt werden.

Die Erfindung betrifft auch ein Auto, welches eine Lüftung mit einem Regelknopf und eine erfindungsgemässe Beduftungsvorrichtung umfasst. Die Beduftungsvorrichtung ist auf dem Regelknopf angeordnet, insbesondere wobei das Halteelement, insbesondere die Vorsprünge, den Regelknopf einklemmt. Insbesondere kann die Beduftungsvorrichtung vom Regelknopf entfernt werden.

Die Anordnung der Beduftungsvorrichtung auf dem Regelknopf hat den Vorteil, dass diese stabil direkt vor der Lüftung montiert werden kann. Die Beduftungsvorrichtung wird angeströmt und die Luft entweicht mit Duftmolekülen aus der Beduftungsvorrichtung.

### Kurze Beschreibung der Zeichnungen

Weitere Ausgestaltungen, Vorteile und Anwendungen der Erfindung ergeben sich aus den abhängigen Ansprüchen und aus der nun folgenden Beschreibung anhand der Figuren. Dabei zeigen:
Fig. 1 eine erfindungsgemässe Beduftungsvorrichtung;
Fig. 2 die Beduftungsvorrichtung beim Austausch des Duftstoffbehälters;
Fig. 3 eine Explosionszeichnung der Beduftungsvorrichtung;
Fig. 4a die Beduftungsvorrichtung mit Sicht von oben und geschlossenen Auslassöffnungen;
Fig. 4b die Beduftungsvorrichtung mit Sicht von oben und geöffneten Auslassöffnungen;
Fig. 5 das Halteelement der Beduftungsvorrichtung; und
Fig. 6 eine stark vereinfachte Schnittansicht durch das Halteelement der Fig. 5.

### Weg zur Ausführung der Erfindung

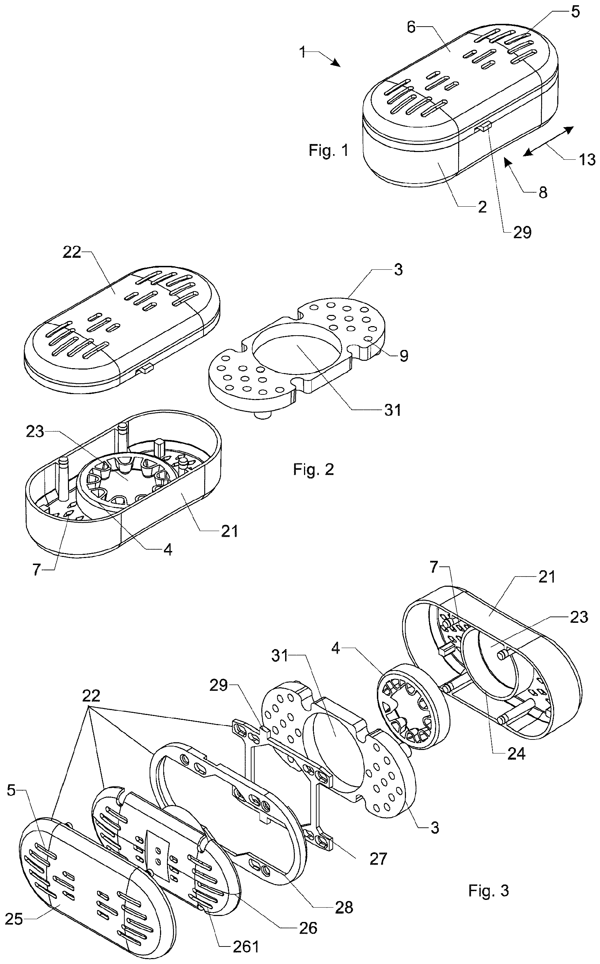

Die Figuren 1 bis 4 zeigen eine erfindungsgemässe Beduftungsvorrichtung 1 für ein Fahrzeug mit einem Gehäuse 2 und einem im Gehäuse 2 angeordneten Duftstoffbehälter 3. Der Duftstoffbehälter 3 ist auswechselbar. Er kann dem Gehäuse 2 entnommen und ein neuer Duftstoffbehälter 3 kann in das Gehäuse 2 eingesetzt werden. Die Beduftungsvorrichtung 1 weist eine Haltevorrichtung mit einem Halteelement 4 auf. Mittels der Haltevorrichtung kann die Beduftungsvorrichtung 1 an einer Lüftung im Innenraum des Fahrzeuges montiert werden.

Die Beduftungsvorrichtung 1 umfasst Auslassöffnungen 5, welche auf einer Auslassseite 6 angeordnet sind. Zudem umfasst die Beduftungsvorrichtung 1 Einlassöffnungen 7, welche auf einer Einlassseite 8 angeordnet sind. Die Einlassseite 8 ist der Auslassseite 6 gegenüberliegend angeordnet.

Ist die Beduftungsvorrichtung 1 an der Lüftung im Innenraum des Fahrzeuges montiert, ist die Einlassseite 8 zur Lüftung gerichtet und die Auslassseite 6 ist von der Lüftung weggerichtet. Luft strömt aus der Lüftung durch die Einlassöffnungen 7, in das Gehäuse 2 und in den im Gehäuse 2 angeordneten Duftstoffbehälter 3. Die Luft kommt mit dem Duftstoff im Duftstoffbehälter 3 in Kontakt, wird mit Duftmolekülen angereichert und verlässt durch die Auslassöffnungen 5 das Gehäuse 2 der Beduftungsvorrichtung 1. Die Luft gelangt in den Innenraum des Fahrzeuges und beduftet den Innenraum.

### Duftstoffbehälter

Der Duftstoffbehälter 3, wie in Fig. 2 dargestellt, ist auswechselbar, wobei der Duftstoffbehälter 3 als Material einen Kompositwerkstoff aus zumindest einem Polymer, insbesondere einem Elastomer, und dem Duftstoff umfasst bzw. aus einem solchen Material besteht. Dabei ist das Polymer mit dem Duftstoff angereichert und fungiert als dessen Träger. Der Duftstift weist bevorzugt mehrere Durchlassöffnungen 9 auf, durch welche die aus dem Lüftungsschlitz austretende Luft strömen kann. Durch das Vorsehen der Durchlassöffnungen 9 wird die Oberfläche vergrössert, an welcher die aus dem Lüftungsschlitz austretende Luft mit dem Duftstoff in Kontakt gerät, was eine erhöhte Anreicherung der die Beduftungsvorrichtung 1 durchströmenden Luft mit Duftstoffmolekülen zur Folge hat. Die Durchlassöffnungen 9 sind vorzugsweise auf die Strömungsrichtung der Luft aus dem Lüftungsschlitz ausgerichtet. Alternativ kann der Duftstoff auch in Form eines Granulats vorgesehen sein, das in einem Käfig enthalten ist.

Wie in Fig. 2 gezeigt, umfasst das Gehäuse 2 eine Gehäusehülle 21 und einen Gehäusedeckel 22. Für den Austausch des Duftstoffbehälters 3 öffnet der Benutzer den Gehäusedeckel 22, tauscht den Duftstoffbehälter 3 aus und schliesst den Duftstoffbehälter 3 durch Schliessen des Gehäusedeckels 22 in das Gehäuse 2 ein.

### Aufbau der Beduftungsvorrichtung

Die Fig. 3 zeigte eine Explosionszeichnung der Beduftungsvorrichtung 1. Wie erwähnt umfasst die Beduftungsvorrichtung 1 eine Gehäusehülle 21. Am Boden der Gehäusehülle 21 sind die Einlassöffnungen 7 angeordnet. Mittig ist eine grössere Gehäuseöffnung 23 vorhanden, welche von einem kreisförmigen Steg 24 umgeben ist. Auf den kreisförmigen Steg 24 ist das bereits erwähnte Halteelement 4 aufspannbar, wobei das Halteelement 4 mittig ebenfalls eine Öffnung 41 aufweist. Das Halteelement 4 umfasst eine in Fig. 3 nicht sichtbare, bodenseitige Nut entlang seines gesamten Umfangs. Ist das Halteelement 4 auf dem kreisförmigen Steg 24 der Gehäusehülle 21 aufgespannt, greift der kreisförmige Steg 24 in die Nut des Halteelements 4.

Zwischen dem Halteelement 4 und den Seitenwänden der Gehäusehülle 21 ist Raum vorhanden, in welchen der Duftstoffbehälter 3 einsetzbar ist. Mittig weist der Duftstoffbehälter 3 eine grössere kreisförmige Öffnung 31 auf. Ist der Duftstoffbehälter 3 im Gehäuse 2 eingesetzt, umgibt der Duftstoffbehälters 3 mit seiner kreisförmigen Öffnung 31 das Halteelement 4. Mit anderen Worten ist das Halteelement 4 innerhalb der kreisförmigen Öffnung 31 des Duftstoffbehälters 3 angeordnet.

Die übrigen in Fig. 3 gezeigten Einzelteile sind Bestandteil des Gehäusedeckels 22. Der Gehäusedeckel 22 umfasst eine Abdeckung 25, eine Schiebevorrichtung 26, ein Griffelement 27 und einen Ring 28. Die Abdeckung 25 und der Ring 28 sind fix miteinander verbunden. Die Schiebevorrichtung 26 und das Griffelement 27 sind ebenfalls fix miteinander verbunden. Bei einer Schiebebewegung bewegen sich die Schiebevorrichtung 26 und das Griffelement 27 relativ zur Abdeckung 25 und zum Ring 28.

An der Abdeckung 25 sind die Auslassöffnungen 5 angeordnet. Diese können mittels der Schiebevorrichtung 26 geöffnet und geschlossen werden. Zum Öffnen und Schliessen der Auslassöffnungen 5 stösst der Benutzer jeweils einen der am Griffelement 27 angeordneten Griffe 29 in den Gehäusedeckel 22 hinein. Das mit der Schiebevorrichtung 26 fix verbundene Griffelement 27 bewegt dadurch die Schiebevorrichtung 26 relativ zur Abdeckung 25.

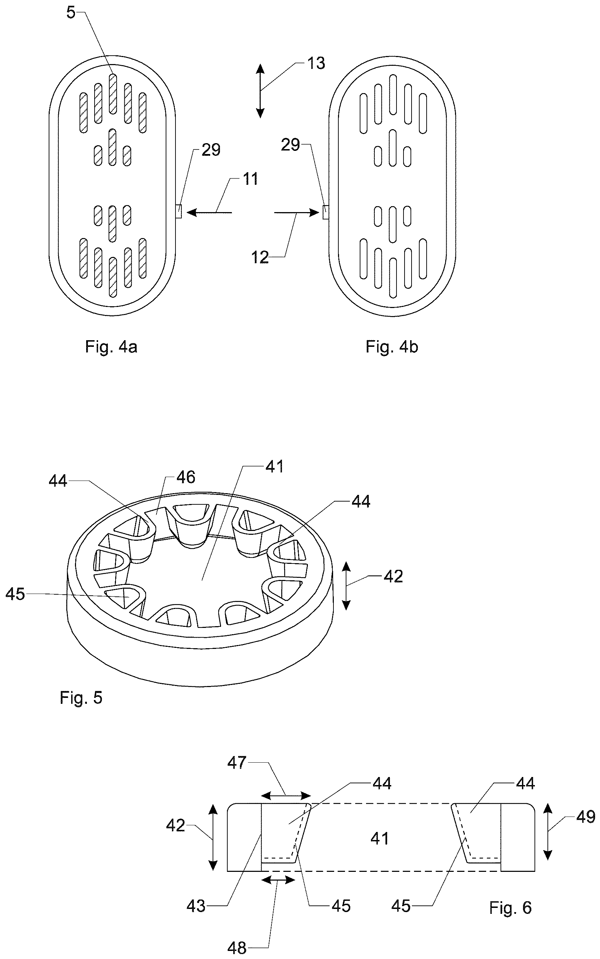

In Fig. 4 sind die möglichen Schiebebewegungen illustriert. Pfeil 11 zeigt auf denjenigen Griff 29, welchen der Benutzer in Pfeilrichtung 11 drücken muss, um die Auslassöffnungen 5 zu öffnen. Pfeil 12 zeigt auf denjenigen Griff 29, welchen der Benutzer in Pfeilrichtung 12 drücken muss, um die Auslassöffnungen 5 zu schliessen.

Im geschlossenen Zustand verdecken die Streben der Schiebevorrichtung 26 die Auslassöffnungen 5. Luft kann die Beduftungsvorrichtung 1 nicht durchströmen. Im offenen Zustand sind die Auslassöffnungen 5 der Abdeckung 25 deckungsgleich mit den Durchlassöffnungen 261 der Schiebevorrichtung 26. Luft kann durch die Beduftungsvorrichtung 1 strömen und mit Duftstoffmolekülen des Duftstoffbehälters 3 angereichert werden.

Die Schiebevorrichtung 26 ist mit einer Arretiervorrichtung ausgestattet. Ist die Schiebevorrichtung 26 arretiert, können die Auslassöffnungen 5 nicht verschlossen oder geöffnet werden. Die Schiebevorrichtung 26 ist blockiert und kann nicht bewegt werden. Die Arretierung erfolgt durch ein Verrasten des Griffelementes 27 am Gehäuse 2. Durch Bewegung eines der Griffe 29 in Richtung des Doppelpfeils 13 kann der Benutzer die Schiebevorrichtung 26 arretieren bzw. die Arretierung lösen.

Die Fig. 5 und 6 zeigen das Halteelement 4 in detaillierter Ansicht. Das Halteelement 4 ist kreisförmig ausgestaltet und umfasst eine zentral angeordnete Öffnung 41. Die Dicke des Halteelements 4 wird als Tiefe 42 bezeichnet.

Die zentral angeordnete Öffnung 41 erstreckt sich über die gesamte Tiefe 42, ist entsprechend durchgehend und wird deshalb als eine Öffnung in Form eines Durchbruchs bezeichnet. Die Tiefe 42 beträgt ca. 8 mm. Wäre die Öffnung 41 nicht durchgehend, sondern von einer Seite verschlossen, wäre sie nicht als Durchbruch, sondern als eine Vertiefung zu bezeichnen. Auch eine solche Ausführung wäre möglich.

Aufgrund der kreisförmigen Öffnung 41 weist der Durchbruch die Form eines Kreiszylinders auf. Ein Kreiszylinder wird durch seine Grundfläche und seine Mantelfläche definiert. Die Grundfläche entspricht der Öffnungsfläche der Öffnung 41. Die Mantelfläche entspricht den Wänden 43 der Öffnung 41. Die Wände 43 umgeben die Öffnung 41 und entsprechen den inneren Seitenwänden des Halteelements 4.

Ausgehend von den Wänden 43 bzw. der Mantelfläche erstrecken sich Vorsprünge 44 in das Innere der Öffnung 41. Insgesamt sind es neun Vorsprünge 44, welche entlang der Mantelfläche äquidistant zueinander angeordnet sind. Die Vorsprünge 44 sind hohl ausgestaltet, wobei der Hohlraum 45 nicht durchgängig, sondern in Form einer Vertiefung ausgestaltet ist. Aufgrund des Hohlraums 45 lassen sich die Vorsprünge sehr gut elastisch deformieren. In Fig. 6 wird der Hohlraum 45 durch gestrichelte Linien illustriert.

Im Weiteren ist zwischen benachbarten Vorsprüngen 44 jeweils ein Zwischenraum 46 vorhanden, d.h. benachbarte Vorsprünge 44 sind voneinander beabstandet. Aufgrund der Zwischenräume 46 können sich die Vorsprünge 44 ebenfalls elastisch gut deformieren.

Fig. 6 zeigt eine Schnittansicht über den Durchmesser des Halteelements 4. Zur Vereinfachung sind lediglich diejenigen zwei Vorsprünge 44 eingezeichnet, welche in der Schnittfläche liegen. Ausgehend vom Rand 43 bzw. von der Mantelfläche des Halteelements 4 ragen die Vorsprünge 44 in das Innere der Öffnung 41. Die Vorsprünge 44 sind dabei derart geformt, dass sie sich entlang der Tiefe 42 unterschiedlich weit in das Innere der Öffnung 41 erstrecken. Beispielsweise erstrecken sich die in Fig. 6 gezeigten Vorsprünge 44 an ihren Oberseiten ausgehend vom Rand 43 über eine Weite 47 in das Innere der Öffnung 41. An ihren Unterseiten erstrecken sich die Vorsprünge 44 allerdings lediglich über eine Weite 48. Somit erstrecken sich die Vorsprünge 44 über unterschiedlich Weiten entlang der Tiefe 42 in das Innere der Öffnung 41.

In der vorliegenden Ausführungsform sind die Vorsprünge 44 derart geformt, dass sie sich ausgehend von der Oberseite zur Unterseite des Halteelements 4 kontinuierlich weniger weit in das Innere der Öffnung 41 erstrecken. Die Erstreckungsweite reduziert sich kontinuierlich. Mit anderen Worten verjüngen sich die Vorsprünge 44 entlang der Tiefe 41.

Entlang der Tiefe 41 erstrecken sich die Vorsprünge 44 über eine Länge 49. Die Länge 49 entspricht dabei etwa 85% der gesamten Tiefe 41 des Halteelements 4.

### Anwendung der Beduftungsvorrichtung

Gesamthaft betrachtet weist die Beduftungsvorrichtung 1 eine Vertiefung auf, welche der Montage der Beduftungsvorrichtung 1 an einer Lüftung in einem Fahrzeug dient. Diese Vertiefung besteht in der vorliegenden Ausführungsform im Wesentlichen aus der Gehäuseöffnung 23 und der Öffnung 41 des Halteelements 4.

Gewisse Lüftungen weisen rundlich geformte Regelknöpfe auf, mit welchen der Benutzer die Lüftungsstärke regulieren kann. Möchte der Benutzer die Beduftungsvorrichtung 1 an die Lüftung montieren, so stülpt er die Beduftungsvorrichtung über einen solchen Regelknopf. Der Regelknopf greift dabei in die Vertiefung der Beduftungsvorrichtung 1. Die Beduftungsvorrichtung 1 klemmt sich mittels den Vorsprüngen 44 des Halteelements 4 am Regelknopf fest.

Während in der vorliegenden Anmeldung bevorzugte Ausführungen der Erfindung beschrieben sind, ist klar darauf hinzuweisen, dass die Erfindung nicht auf diese beschränkt ist und in auch anderer Weise innerhalb des Umfangs der folgenden Ansprüche ausgeführt werden kann.

## Patentansprüche

1. Beduftungsvorrichtung (1) für ein Fahrzeug umfassend
- ein Gehäuse (2),
- einen im Gehäuse (2) angeordneten Duftstoffbehälter (3),
- eine Haltevorrichtung zum Anbringen der Beduftungsvorrichtung (1) an einer Lüftung im Innenraum des Fahrzeuges,
wobei die Haltevorrichtung ein Halteelement (4) mit einer Öffnung (41) aufweist und die Öffnung (41) in Form eines Durchbruchs oder einer Vertiefung ausgestaltet ist und eine Tiefe (42) aufweist,
**dadurch gekennzeichnet, dass** am Rand (43) bzw. an den Wänden der Öffnung (41) Vorsprünge (44) angeordnet sind, wobei sich die Vorsprünge (44) entlang der Tiefe (42) unterschiedlich weit in das Innere der Öffnung (41) erstrecken, insbesondere entlang der Tiefe (42) verjüngen.

2. Beduftungsvorrichtung nach Anspruch 1, wobei der Durchbruch oder die Vertiefung die Form eines Zylinders, insbesondere eines Kreiszylinders oder eines Zylinders mit einer elliptischen Grundfläche, mit einer Mantelfläche und einer Höhe aufweist, wobei die Vorsprünge (44) an der Mantelfläche angeordnet sind und sich entlang der Höhe unterschiedlich weit in das Innere des Zylinders erstrecken.

3. Beduftungsvorrichtung nach einem der vorangehenden Ansprüche, wobei die Vorsprünge (44) hohl sind,
insbesondere mit einem Hohlraum (45), welcher in Form einer Vertiefung und nicht in Form eines Durchbruchs ausgestaltet ist.

4. Beduftungsvorrichtung nach einem der vorangehenden Ansprüche, wobei die einzelnen Vorsprünge (44) voneinander beabstandet sind, insbesondere mittels eines Abstands beabstandet, wobei der Abstand grösser als ein Drittel der Breite des Vorsprungs (44) ist.

5. Beduftungsvorrichtung nach einem der vorangehenden Ansprüche, wobei die Öffnung (41) eine Tiefe (42) von mindestens 5 mm, insbesondere mindestens 7.5 mm aufweist.

6. Beduftungsvorrichtung nach einem der vorangehenden Ansprüche, wobei sich die Vorsprünge (44) entlang der Tiefe (42) über mindestens 50%, insbesondere mindestens 75%, der Tiefe erstrecken.

7. Beduftungsvorrichtung nach einem der vorangehenden Ansprüche, wobei die Haltevorrichtung eine die Öffnung (41) umgebende Nut, insbesondere eine kreisförmige Nut, aufweist, insbesondere geeignet, um die Haltevorrichtung am Gehäuse (2) zu befestigen.

8. Beduftungsvorrichtung nach einem der vorangehenden Ansprüche, aufweisend eine Schiebevorrichtung (26) mit welcher Einlassöffnungen (7) und/oder Auslassöffnungen (5) des Gehäuses (2) mittels einer Schiebebewegung zumindest teilweise verschliessbar sind.

9. Beduftungsvorrichtung nach Anspruch 8, wobei die Schiebevorrichtung (26) mindestens zwei Griffe (29) aufweist, durch dessen Betätigung die Schiebbevorrichtung (26) verschiebbar ist,
insbesondere wobei die Betätigung durch Hineindrücken der Griffe (29) in das Gehäuse (2) erfolgt.

10. Beduftungsvorrichtung nach einem der Ansprüche 8 oder 9, wobei die Schiebevorrichtung (26) eine Arretiervorrichtung aufweist, derart dass in arretierter Stellung die Griffe (29) nicht betätigbar und die Schiebebewegung nicht durchführbar sind.

11. Beduftungsvorrichtung nach einem der vorangehenden Ansprüche, wobei der Duftstoffbehälter (3),
- das Halteelement (4) umgibt, und/oder
- eine Öffnung (31) aufweist, innerhalb welcher das Halteelement (4) angeordnet ist,
insbesondere wobei die Beduftungsvorrichtung (1) ausgestaltet ist, genau einen Duftstoffbehälter (3) im Gehäuse (2) aufzunehmen.

12. Beduftungsvorrichtung nach einem der vorangehenden Ansprüche, aufweisend eine Vertiefung (23, 41), geeignet, dass bei Anbringung der Beduftungsvorrichtung (1) an der Lüftung ein Regelknopf der Lüftung in die Vertiefung (23, 41) greifen kann.

13. Beduftungsvorrichtung nach Anspruch 12, wobei die Vorsprünge (44) in die Vertiefung (23, 41) der Beduftungsvorrichtung (1) hineinragen, geeignet, um den Regelknopf einzuklemmen.

14. Auto, aufweisend eine Lüftung mit einem Regelknopf und einer Beduftungsvorrichtung (1) nach einem der vorangehenden Ansprüche, wobei die Beduftungsvorrichtung (1) auf dem Regelknopf angeordnet ist, insbesondere wobei das Halteelement (4), insbesondere die Vorsprünge (44), den Regelknopf einklemmt.

15. Auto nach Anspruch 14, wobei die Beduftungsvorrichtung (1) vom Regelknopf entfernbar ist.

## Claims

1. Scenting device (1) for a vehicle comprising
- a housing (2),
- a scent container (3) arranged in the housing (2),
- a holding device for attaching the scenting device (1) to a ventilation system in the interior of the vehicle,
wherein the holding device has a holding element (4) with an opening (41) and the opening (41) is designed in the form of an aperture or a recess and has a depth (42),
**characterised in that** projections (44) are arranged on the edge (43) or on the walls of the opening (41), wherein the projections (44) extend into the interior of the opening (41) to different extents along the depth (42), in particular tapering along the depth (42).

2. Scenting device according to claim 1,
wherein the aperture or the recess has the shape of a cylinder, in particular a circular cylinder or a cylinder with an elliptical base, with a lateral surface and a height, wherein the projections (44) are arranged on the lateral surface and extend along the height to different extents into the interior of the cylinder.

3. Scenting device according to one of the preceding claims, wherein the projections (44) are hollow,
in particular with a cavity (45) which is designed in the form of a depression and not in the form of an aperture.

4. Scenting device according to one of the preceding claims, wherein the individual projections (44) are spaced apart from each other, in particular spaced apart by means of a distance, wherein the distance is greater than one third of the width of the projection (44) .

5. Scenting device according to one of the preceding claims, wherein the opening (41) has a depth (42) of at least 5 mm, in particular at least 7.5 mm.

6. Scenting device according to one of the preceding claims, wherein the projections (44) extend along the depth (42) over at least 50%, in particular at least 75%, of the depth.

7. Scenting device according to one of the preceding claims, wherein the holding device comprises a groove, in particular a circular groove, surrounding the opening (41), in particular suitable for fastening the holding device to the housing (2).

8. Scenting device according to one of the preceding claims, comprising a sliding device (26) with which inlet openings (7) and/or outlet openings (5) of the housing (2) can be at least partially closed by means of a sliding movement.

9. Scenting device according to claim 8,
wherein the sliding device (26) has at least two handles (29), by the actuation of which the sliding device (26) is displaceable,
in particular wherein the actuation is effected by pressing the handles (29) into the housing (2).

10. Scenting device according to one of claims 8 or 9, wherein the sliding device (26) comprises a locking device such that in the locked position the handles (29) cannot be actuated and the sliding movement cannot be performed.

11. Scenting device according to one of the preceding claims, wherein the scent container (3),
- surrounds the holding element (4), and/or
- has an opening (31) within which the holding element (4) is arranged,
in particular wherein the scenting device (1) is configured to receive exactly one scent container (3) in the housing (2).

12. Scenting device according to one of the preceding claims, comprising a recess (23, 41), suitable for allowing a control knob of the ventilation to engage in the recess (23, 41) when the scenting device (1) is attached to the ventilation.

13. Scenting device according to claim 12,
wherein the projections (44) project into the recess (23, 41) of the scenting device (1), suitable for clamping the control knob.

14. Car, comprising a ventilation with a control knob and a scenting device (1) according to one of the preceding claims, wherein the scenting device (1) is arranged on the control knob, in particular wherein the retaining element (4), in particular the projections (44), clamps the control knob.

15. car according to claim 14, wherein the scenting device (1) is removable from the control knob.

## Revendications

1. Dispositif de diffusion de parfum (1) pour un véhicule, comprenant
- un boîtier (2),
- un réservoir de parfum (3) disposé dans le boîtier (2),
- un dispositif de support pour fixer le dispositif de diffusion de parfum (1) à une ventilation dans l'habitacle du véhicule,
le dispositif de support présentant un élément de support (4) avec une ouverture (41) et l'ouverture (41) étant conçue sous la forme d'un passage ou d'une cavité et présentant une profondeur (42),
**caractérisé en ce que** des protubérances (44) sont disposées sur le bord (43) ou sur les parois de l'ouverture (41), les protubérances (44) s'étendant à l'intérieur de l'ouverture (41) sur une distance différente le long de la profondeur (42), en particulier en se rétrécissant le long de la profondeur (42).

2. Dispositif de diffusion de parfum selon la revendication 1, dans lequel le passage ou la cavité présente la forme d'un cylindre, en particulier d'un cylindre circulaire ou d'un cylindre avec une surface de base elliptique, avec une surface d'enveloppe et une hauteur, les protubérances (44) étant disposées sur la surface d'enveloppe et s'étendant le long de la hauteur à des distances différentes à l'intérieur du cylindre.

3. Dispositif de diffusion de parfum selon l'une des revendications précédentes, dans lequel les protubérances (44) sont creuses,
en particulier avec un espace creux (45) qui est configuré sous la forme d'une cavité et non d'un passage.

4. Dispositif de diffusion de parfum selon l'une des revendications précédentes, dans lequel les différentes protubérances (44) sont espacées les unes des autres, notamment au moyen d'une distance, la distance étant supérieure à un tiers de la largeur de la protubérance (44) .

5. Dispositif de diffusion de parfum selon l'une des revendications précédentes, dans lequel l'ouverture (41) présente une profondeur (42) d'au moins 5 mm, en particulier d'au moins 7,5 mm.

6. Dispositif de diffusion de parfum selon l'une des revendications précédentes, dans lequel les protubérances (44) s'étendent le long de la profondeur (42) sur au moins 50%, notamment au moins 75% de la profondeur.

7. Dispositif de diffusion de parfum selon l'une des revendications précédentes, dans lequel le dispositif de support comporte une rainure, notamment circulaire, entourant l'ouverture (41), notamment adaptée pour fixer le dispositif de support au boîtier (2).

8. Dispositif de diffusion de parfum selon l'une des revendications précédentes, présentant un dispositif coulissant (26) avec lequel des ouvertures d'entrée (7) et/ou des ouvertures de sortie (5) du boîtier (2) peuvent être fermées au moins partiellement au moyen d'un mouvement de coulissement.

9. Dispositif de diffusion de parfum selon la revendication 8, dans lequel le dispositif coulissant (26) présente au moins deux poignées (29) dont l'actionnement permet de faire coulisser le dispositif coulissant (26),
en particulier, l'actionnement s'effectuant en enfonçant les poignées (29) dans le boîtier (2).

10. Dispositif de diffusion de parfum selon l'une des revendications 8 ou 9, dans lequel le dispositif coulissant (26) comporte un dispositif de blocage de telle sorte qu'en position bloquée, les poignées (29) ne peuvent pas être actionnées et le mouvement de coulissement ne peut pas être effectué.

11. Dispositif de diffusion de parfum selon l'une des revendications précédentes, dans lequel le réservoir de parfum (3),
- entoure l'élément de support (4), et/ou
- présente une ouverture (31) à l'intérieur de laquelle est disposé l'élément de support (4),
en particulier dans lequel le dispositif de diffusion de parfum (1) est conçu pour recevoir exactement un récipient de parfum (3) dans le boîtier (2).

12. Dispositif de diffusion de parfum selon l'une des revendications précédentes, comprenant une cavité (23, 41) adaptée pour que, lorsque le dispositif de diffusion de parfum (1) est monté sur la ventilation, un bouton de réglage de la ventilation puisse s'engager dans la cavité (23, 41).

13. Dispositif de diffusion de parfum selon la revendication 12, dans lequel les protubérances (44) font saille dans la cavité (23, 41) du dispositif de diffusion de parfum (1), adapté pour pincer le bouton de réglage.

14. Voiture comportant une ventilation avec un bouton de réglage et un dispositif de diffusion de parfum (1) selon l'une des revendications précédentes, le dispositif de diffusion de parfum (1) étant disposé sur le bouton de réglage, notamment l'élément de soutien (4), notamment les protubérances (44), pinçant le bouton de réglage.

15. Voiture selon la revendication 14, dans laquelle le dispositif de diffusion de parfum (1) peut être retiré du bouton de réglage.
